# EUROPÄISCHE PATENTSCHRIFT

(11) **EP 1 641 439 B1**
(45) Veröffentlichungstag und Bekanntmachung des Hinweises auf die Patenterteilung: **17.01.2007**
(21) Anmeldenummer: 04739854.0
(22) Anmeldetag: 14.06.2004
(51) Int. Cl.: A61K 9/20

(54) **TABLETTEN ENTHALTEND ENROFLOXACIN UND GESCHMACK- UND/ODER AROMASTOFFE**
TABLETS CONTAINING ENROFLOXACIN AND FLAVOURING AGENTS AND/OR FLAVOURS
COMPRIMES CONTENANT DE L'ENROFLOXACINE ET DES AGENTS AROMATISANTS ET/OU DES AROMES

(30) Priorität: 26.06.2003 DE 10328666
(43) Veröffentlichungstag der Anmeldung: 05.04.2006
(73) Patentinhaber: Bayer HealthCare AG, 51368 Leverkusen (DE)
(72) Erfinder: KANIKANTI, Venkata-Rangarao, 51381 Leverkusen (DE); BONGAERTS, Sabine, 51379 Leverkusen (DE)
(86) Internationale Anmeldenummer: PCT/EP2004/006370
(87) Internationale Veröffentlichungsnummer: WO 2005/000275

(56) Entgegenhaltungen:
- WO-A-94/02144
- US-A- 5 152 986
- US-A- 5 808 076

## Beschreibung

Die vorliegende Erfindung betrifft Tabletten für Tiere, die Enrofloxacin sowie Geschmacks- und/oder Aromastoffe enthalten.

Die Verabreichung von Tabletten an Tiere ist problematisch, da diese für die Tiere ohne jede Attraktivität sind und von ihnen in der Regel nur unfreiwillig aufgenommen werden. Üblicherweise müssen die Tabletten in Futter verpackt werden, um sie zu applizieren. Hierbei ist nicht immer garantiert, dass die Arznei vollständig und damit in der richtigen Dosierung appliziert werden kann. Auch das Freisetzungsprofil des Arzneimittels kann sich bei der Gabe im Futter ändern.

Es ist im Prinzip bereits bekannt, dass die Palatabilität durch Zugabe geeigneter Aromen und/oder Geschmacksstoffe gesteigert werden kann. Allerdings werden durch diesen Zusatz die mechanischen Eigenschaften der Tabletten häufig in einem in der Praxis nicht akzeptablen Maß verschlechtert.

In US-A-5 808 076 wird eine wohlschmeckende Tablette beschrieben, welche Enrofloxacingranulate mit weiteren Hilfstoffen wie Maisstärke und mikrokristalline Cellulose enthält.

Es besteht daher Bedarf an gut palatablen Tabletten mit akzeptablen mechanischen Eigenschaften.

Die Erfindung betrifft:
Tabletten enthaltend:
20 bis 45 Gew. % Enrofloxacin
18 bis 35 Gew. % Lactose
5 bis 10 Gew. % Mikrokristalline Cellulose und
5 bis 20 Gew. % Fleischaroma.

Die Angaben in Gewichtsprozent sind bezogen auf das Gesamtgewicht der Tablette.

Enrofloxacin wird eingesetzt in einer Menge von 20 bis 45 Gew. %, bevorzugt 23 bis 42 Gew. %.

Enrofloxacin trägt die systematische Bezeichnung 1-Cyclopropyl-7-(4-ethyl-1-piperazinyl)-6-fluor-1,4-dihydro-4-oxo-3-chinolincarbonsäure und hat die folgende Strukturformel:

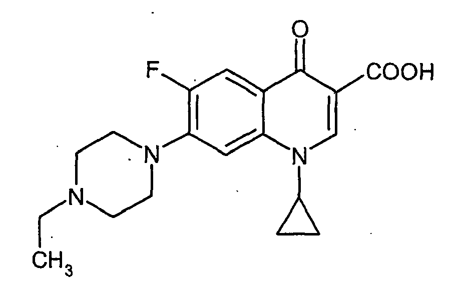

Erfindungsgemäß kann Enrofloxacin auch in Form seiner pharmazeutisch verwendbaren Salze und Hydrate eingesetzt werden.

Als Salze kommen pharmazeutisch verwendbare Säureadditionssalze und basische Salze in Frage.

Als pharmazeutisch verwendbare Salze sind beispielsweise die Salze der Salzsäure, Schwefelsäure, Essigsäure, Glykolsäure, Milchsäure, Bernsteinsäure, Zitronensäure, Weinsäure, Methansulfonsäure, 4-Toluolsulfonsäure, Galacturonsäure, Gluconsäure, Embonsäure, Glutaminsäure oder Asparaginsäure zu verstehen. Ferner lässt sich Enrofloxacin auch an saure oder basische Ionenaustauscher binden. Als pharmazeutisch verwendbare basische Salze seien die Alkalisalze, beispielsweise die Natrium- oder Kaliumsalze, die Erdalkalisalze, beispielsweise die Magnesium-, oder Calciumsalze; die Zinksalze, die Silbersalze und die Guanidiniumsalze genannt.

Unter Hydraten werden sowohl die Hydrate des Enrofloxacins selbst als auch die Hydrate seiner Salze verstanden.

Lactose ist ein handelsüblicher Arzneimittelhilfsstoff, der in verschiedenen Formen erhältlich ist, z.B. sprühgetrocknet oder als wasserfreie Lactose. Bevorzugt wird erfindungsgemäß Lactose-Monohydrat eingesetzt (z.B. Milchzucker fein der Fa. DMV International). Die erfindungsgemäßen Tabletten enthalten 18 bis 35 Gew. % Lactose, bevorzugt 19 bis 30 Gew. % bezogen auf das Gesamtgewicht der Tablette.

Mikrokristalline Cellulose ist ein handelsüblicher Arzneimittelhilfsstoff (z. B. Avicel^{®} PH 101 der Fa. FMC). Die erfindungsgemäßen Tabletten enthalten 5 bis 10 Gew. %, bevorzugt 5,5 bis 8 Gew. % bezogen auf das Gesamtgewicht der Tablette.

Als Fleischaroma sind Trockenleberpulver aus Rind, Geflügel, Schaf oder Schwein bevorzugt aus Geflügel und Schwein, sowie andere Aromenzubereitungen geeignet. Ganz besonders geeignet sind die unter den Bezeichnungen Artificial Beef Flavor und BAYOPAL^{®} im Handel befindlichen Aromen der Firmen Pharma Chemie (Artificial Beef Flavor) und Haarmann und Reimer (BAYOPAL^{®}).

Das Fleischaroma wird vorzugsweise in einer Menge von 5 % bis 20 %, bevorzugt 7 % bis 15 %, besonders bevorzugt 9 % bis 11 % eingesetzt. Die Prozentangaben sind dabei Gewichtsprozent der fertigen Tablette.

Zusätzlich zu den oben angegebenen Inhaltsstoffen können die erfindungsgemäßen Tabletten auch weitere übliche pharmazeutische Träger- und Hilfsstoffe enthalten.

Als Trägerstoffe seien genannt alle physiologisch verträglichen festen Inertstoffe. Alle solche dienen anorganische und organische Stoffe. Anorganische Stoffe sind z.B. Kochsalz, Carbonate wie Calciumcarbonat, Hydrogencarbonate, Aluminiumoxide, Kieselsäuren, Tonerden, gefälltes oder kolloidales Siliciumdioxid, Phosphate.

Bevorzugt enthalten die erfindungsgemäßen Tabletten Siliciumdioxid, insbesondere kolloidales wasserfreies Siliciumdioxid, in Mengen von 0,05 bis 0,3 Gew. % insbesondere, 0,1 bis 0,2 Gew. % bezogen auf das Gesamtgewicht der Tablette.

Organische Stoffe sind z.B. Zucker, Zellulose, Nahrungs- und Futtermittel wie Milchpulver, Tiermehle, Getreidemehle und -schrote, Stärken.

Bevorzugt enthalten die erfindungsgemäßen Tabletten Stärke, wie z. B. Maisstärke, als weiteren Trägerstoff, und zwar in Mengen von üblicherweise 10 bis 40 Gew. %, vorzugsweise 15 bis 30 Gew. %, besonders bevorzugt 18 bis 26 Gew. % bezogen auf das Gesamtgewicht der Tablette.

Die Tabletten können weitere übliche pharmazeutische Hilfsstoffe enthalten. Als solche seien beispielhaft genannt: Schmier- und Gleitmittel wie z.B. Magnesiumstearat, Stearinsäure, Talkum, Bentonite; zerfallsfördernde Substanzen wie Stärke, quervernetzte Natrium-Carboxymethylcellulose oder quervernetztes Polyvinylpyrrolidon; Bindemittel wie z.B. Stärke, Gelatine, Celluloseether oder lineares Polyvinylpyrrolidon sowie Trockenbindemittel wie mikrokristalline Cellulose.

Bevorzugt enthalten die erfindungsgemäßen Tabletten ein Schmiermittel, insbesondere Magnesiumstearat, in Mengen von 0,4 bis 1,0 Gew. %, bevorzugt 0,5 bis 0,8 Gew. % bezogen auf das Gesamtgewicht der Tablette.

Bevorzugt enthalten die erfindungsgemäßen Tabletten ein Bindemittel, insbesondere ein Polyvinylpyrrolidon (z.B. Polyvidone), in Mengen von 1,5 bis 4 Gew. %, bevorzugt 2 bis 3 Gew. % bezogen auf das Gesamtgewicht der Tablette.

Die erfindungsgemäßen Tabletten lassen sich herstellen nach einem Verfahren, bei dem man
(a) Enrofloxacin, Lactose, gegebenenfalls Fleischaroma sowie gegebenenfalls weitere Hilfsstoffe mischt,
(b) die Mischung unter Zusatz von Wasser oder wässriger Lösungen weiterer Hilfsstoffe granuliert
(c) diese Mischung trocknet,
(d) nach Trocknung mikrokristalline Cellulose und gegebenenfalls weitere Hilfsstoffe sowie Fleischaroma, sofern dieses noch nicht in Schritt (a) zugegeben wurde, zusetzt,
(e) und die Mischung anschließend zu Tabletten verpresst.

In Schritt (a) wird als weiterer Hilfsstoff vorzugsweise Stärke, insbesondere Maisstärke zugesetzt. Besonders günstig ist es hier nur einen Teil der insgesamt eingesetzten Stärkemenge zuzusetzen.

In Schritt (b) wird als weiterer Hilfsstoff vorzugsweise eine wässrige Polyvinylpyrrolidon-Lösung zugesetzt.

Bei der Trocknung in Schritt (c) erweist es sich als vorteilhaft eine Restfeuchte von unter 5 % vorzugsweise 1 bis 4 % (bestimmt als Trocknungsverlust) einzuhalten.

In Schritt (d) werden als weitere Hilfsmittel vorzugsweise Stärke, kolloidales Siliziumdioxid und Magnesiumstearat zugegeben. Sofern bereits ein Teil der Stärke in Schritt (a) zugesetzt wurde, wird in Schritt (d) der zweite Teil der Gesamtmenge zugemischt.

Das antibiotische Wirkungsspektrum von Enrofloxacin ist bekannt. Die erfindungsgemäßen Arzneimittel eignen sich daher zur Prophylaxe und Behandlung entsprechender bakterieller Erkrankungen und Erkrankungen, die durch bakterienähnliche Organismen hervorgerufen werden. Die erfindungsgemäßen Mittel eignen sich generell zur Anwendung in der Tierhaltung und Tierzucht bei Nutz-, Zucht-, Zoo-, Labor-, Versuchs- und Hobbytieren. Vorzugsweise werden sie natürlich bei solchen Tieren eingesetzt, bei denen eine Verbesserung der Palatabilität durch den Fleischaromazusatz zu erwarten ist.

Zu den Nutz- und Zuchttieren gehören Säugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär.

Zu Labor- und Versuchstieren gehören Mäuse, Ratten, Meerschweinchen, Goldhamster, Hunde und Katzen.

Zu den Hobbytieren gehören Hunde und Katzen.

Die erfindungsgemäßen Mittel werden besonders bevorzugt bei Hunden und Katzen, insbesondere Hunden eingesetzt.

Zu den bakteriellen Erkrankungen bei Tieren zählen z.B. Schweinedysenterie; Leptospirose bei Rind, Schwein, Pferd, Hund; Campylobacter-Enteritis beim Rind; Camylobacter-Abort bei Schaf und Schwein; Infektionen der Haut; Pyodermien beim Hund; Otitis extema; Mastitis des Rindes, der Schafe und der Ziege; Streptokokkenmastitis; Streptokokkeninfektion des Pferdes, beim Schwein und anderen Tierarten; Pneumokokkeninfektion des Kalbs, und bei anderen Tierarten; Malleus; Konjunktivitis; Enteritiden; Pneumonien; Brucellose bei Ring, Schaf, Schwein; Rhinitis atrophicans des Schweins; Salmonellose bei Rind, Pferd, Schaf, und anderen Tierarten; Septikämien; Escherichia coli Infektion beim Ferkel; Metritis-Mastitis-Agalaktic-(MMA)-Syndrom; Klebsiella Infektionen; Pseudotuberkulose; Infektiöse Pleuropneumonie; Primäre Pasteureliosen; Fohlenlähme; Nektrobazillose beim Rind und bei Haustieren; Leptospirose; Rotlauf des Schweins und anderen Tierarten, Listeriose; Milzbrand; Clostridiosen; Tetranusinfektionen, Botulismus; Infektionen mit Corynebacterium pyogenes; Tuberkulose beim Rind, Schwein, und anderen Tierarten; Paratuberkulose der Wiederkäuer; Nokardiose; Q-Fieber; Ornithose-Psittakose; Enzephalomycelitis; Mykoplasmose des Rindes und anderer Tiere, Enzootische Pneumonie der Schweine.

Die erfindungsgemäßen Tabletten haben eine vergleichsweise geringe Härte (z. B. hat die Tablette gemäß Beispiel (1) einen Durchmesser von 5 mm und eine Härte in der Größe von 20-30 N), dies ist bei Tabletten mit Aromazusatz ein bekanntes Problem. Überraschenderweise zeichnen sich die erfindungsgemäßen Tabletten durch eine im Vergleich zu ihrer geringen Härte relativ hohe Abriebfestigkeit aus, so dass sie in der Praxis gut einsetzbar sind. In den Arzneibüchern bzw. Pharmakopöen (z. B. Ph. Eur. oder USP) werden Testverfahren und Mindestanforderungen für die Abriebfestigkeit von Tabletten beschrieben.

### Beispiele

| **Inhaltsstoffe** | **(1) mg** | **(2) mg** | **(3) mg** |
|---|---|---|---|
| Enrofloxacin | 15,00 | 50,00 | 150,00 |
| Lactose-Monohydrat | 17,80 | 23,60 | 100,40 |
| Maisstärke | 15,20 | 22,40 | 86,10 |
| Mikrokristalline Cellulose | 4,00 | 8,00 | 28,00 |
| Polyvidone | 1,50 | 3,00 | 10,00 |
| Magnesiumstearat | 0,40 | 0,80 | 2,80 |
| Wasserfreies kolloidales Siliziumdioxid | 0,10 | 0,20 | 0,70 |
| Künstl. Rindfleischaroma, bestrahlt (irradiated artificial beef flavor) | 6,00 | 12,00 | 42,00 |
| Tablettengewicht | 60,00 | 120,00 | 420,00 |

## Patentansprüche

1. Tabletten enthaltend:
20 bis 45 Gew. % Enrofloxacin
18 bis 35 Gew. % Lactose
5 bis 10 Gew. % Mikrokristalline Cellulose und
5 bis 20 Gew. % Fleischaroma.

2. Verfahren zur Herstellung der Tabletten gemäß Anspruch 1, bei dem man
(a) Enrofloxacin, Lactose, gegebenenfalls Fleischaroma sowie gegebenenfalls weitere Hilfsstoffe mischt,
(b) die Mischung unter Zusatz von Wasser oder wässriger Lösungen weiterer Hilfsstoffe granuliert
(c) diese Mischung trocknet,
(d) nach Trocknung mikrokristalline Cellulose und gegebenenfalls weitere Hilfsstoffe sowie Fleischaroma, sofern dieses noch nicht in Schritt (a) zugegeben wurde, zusetzt,
(e) und die Mischung anschließend zu Tabletten verpresst.

## Claims

1. Tablets comprising:
from 20 to 45% by weight of enrofloxacin
from 18 to 35% by weight of lactose
from 5 to 10% by weight of microcrystalline cellulose, and
from 5 to 20% by weight of meat flavour.

2. Process for producing tablets according to Claim 1, in which
(a) enrofloxacin, lactose, where appropriate meat flavour and also, where appropriate, additional adjuvants are mixed,
(b) the mixture is granulated in the added presence of water or aqueous solutions of additional adjuvants,
(c) this mixture is dried,
(d) after drying, microcrystalline cellulose and, where appropriate, additional adjuvants and also meat flavour, provided this was not added in step (a), are admixed,
(e) and the mixture is subsequently pressed into tablets.

## Revendications

1. Comprimés contenant :
20 à 45 % en poids d'enrofloxacine,
18 à 35 % en poids de lactose,
5 à 10 % en poids de cellulose microcristalline, et
5 à 20 % en poids d'aromatisant à odeur de viande.

2. Procédé de fabrication des comprimés suivant la revendication 1, dans lequel
(a) on mélange l'enrofloxacine, le lactose, éventuellement l'aromatisant à odeur de viande ainsi que, le cas échéant, d'autres substances auxiliaires,
(b) on granule le mélange en y ajoutant de l'eau ou des solutions aqueuses d'autres substances auxiliaires,
(c) on sèche le mélange,
(d) après le séchage, on ajoute la cellulose microcristalline et, le cas échéant, d'autres substances auxiliaires ainsi que l'aromatisant à odeur de viande, dans la mesure où ce dernier n'a pas encore été ajouté dans l'étape (a),
(e) et on presse ensuite le mélange en comprimés.
